# EUROPEAN PATENT APPLICATION

(11) **EP 4 279 069 A1**
(43) Date of publication of application: **22.11.2023**
(21) Application number: 22315110.1
(22) Date of filing: 20.05.2022
(51) Int. Cl.: A61K 31/167, A61K 31/395, A61P 1/16, A61P 29/00, A61P 39/00

(54) **A PHARMACEUTICAL COMPOSITION FOR USE IN PREVENTING AND/OR REDUCING HEPATOTOXICITY INDUCED BY ACETAMINOPHEN**

(71) Applicant: APTYS Pharmaceuticals SAS, 63360 Saint Beauzire (FR)
(72) Inventor: BOUTIGNON, François, 63000 Clermont-Ferrand (FR); MALLET, Christophe, 63540 Romagnat (FR); ESCHALIER, Alain, 63400 Chamalières (FR)
(74) Representative: Holme Patent A/S

(57) **Abstract**

The present invention relates to a pharmaceutical composition for use in preventing and/or reducing hepatotoxicity induced by acetaminophen. Said pharmaceutical composition comprises a combination of acetaminophen and nefopam, as nefopam effectively reduces the hepatotoxicity of acetaminophen.

## Description

The present invention relates to a pharmaceutical composition for use in preventing and/or reducing hepatotoxicity induced by acetaminophen, while improving acetaminophen's analgesic effect by using a bimodal strategy.

Acetaminophen, (also known as paracetamol, N-acetyl-p-aminophenol or APAP) is a commonly used mild analgesic and antipyretic available in numerous prescriptions and over the counter formulations. It is considered a first line analgesic for many patients, primarily due to concern about the side effects of non-steroidal anti-inflammatory (NSAID) and opioid-derived agents.

However, acetaminophen has long been known to cause liver toxicity when administered at high dosages. Acetaminophen toxicity is an ongoing global problem that continues to result in cases of hepatotoxicity, acute liver failure, and even irreversible liver injury necessitating liver transplantation and/or resulting in death.

Acetaminophen has a high bioavailability, with almost 80% of the drug being absorbed when taken orally. In individuals without liver injury, the half-life of acetaminophen is roughly 2-3 h.

At therapeutic doses, about 95% of acetaminophen undergoes detoxification in the liver and is excreted in the urine as glucuronate or sulfate conjugates. About 5% or less is metabolized through the activity of CYPs (primarily CYP2E) to N-acetyl-p-benzoquinoneimine (NAPQI), a highly reactive metabolite. NAPQI is normally conjugated with glutathione, but when acetaminophen is taken in large doses, unconjugated NAPQI accumulates and causes hepatocellular injury, leading to centrilobular necrosis that may progress to liver failure.

The injury produced by NAPQI involves two mechanisms: (1) covalent binding to hepatic proteins, which causes damage to cellular membranes and mitochondrial dysfunction, and (2) depletion of glutathione, making hepatocytes more susceptible to reactive oxygen species (ROS) induced injury. Because alcohol induces CYP2E in the liver, toxicity can occur at lower doses in chronic alcoholics.

Hepatic necrosis occurs only when glutathione concentrations fall below a critical level, resulting in excess NAPQI, which triggers a cascade of cellular events resulting in necrotic cell death in the liver. The only approved agent on the market for treatment of acetaminophen overdose is the cysteine donor N-acetylcysteine (NAC), which serves as a precursor for glutathione i.e.,stimulates hepatic synthesis of glutathione and hence accelerates the detoxification of NAPQI.

Early diagnosis, modern medicine and use of NAC have considerably improved outcomes for patients who present early with toxicity. However, there is a demand for providing new acetaminophen treatment regimes and compositions which prevents the onset of hepatotoxicity associated with acetaminophen irrespectively of the administered dosage.

Thus, it is a first aspect of the present invention to provide a pharmaceutical composition comprising acetaminophen, and wherein the acetaminophen can be administered in a higher dosage without initiating hepatotoxicity and/or reducing the hepatotoxic risk.

It is a second aspect of the present invention to provide a pharmaceutical composition comprising acetaminophen for use in preventing and/or reducing hepatotoxicity induced by acetaminophen.

It is a third aspect of the present invention to provide a multimodal analgesia that comprises acetaminophen and which can be administered orally.

These and further aspects are achieved according to the present invention by providing a pharmaceutical composition for use in preventing and/or reducing hepatotoxicity induced by acetaminophen, and wherein said composition comprises a combination of acetaminophen and nefopam.

The inventors of the present invention have surprisingly discovered that nefopam effectively reduces the hepatotoxicity of acetaminophen, thus by providing a composition comprising acetaminophen and nefopam, acetaminophen can be safely administered, also at relatively high dosages.

Without being bound by theory, it is assumed that nefopam somehow prevents the depletion of glutathione in hepatocytes and/or reduces the oxidative stress, mitochondrial dysfunction, DNA damage and hepatic injury caused by acetaminophen. However, further experiments are required in order to evaluate the exact protective mechanism of nefopam on acetaminophen.

Nefopam (5-methyl-1-phenyl-1,3,4,6-tetrahydro-2,5-benzoxazo-cine) is an analgesic that has been used to treat mild to moderate postoperative pain in different clinical settings. It is a non-opioid and non-steroidal drug that is chemically distinct and pharmacologically unrelated to any other known analgesics. (Heel et al., Nefopam: A review of its pharmacological properties and therapeutic efficacy. Drugs 1980; 19: 249-67).

Pharmaceutical compositions comprising both nefopam and acetaminophen are known in the art in which nefopam has been co-administrated with acetaminophen in order to provide a 'multimodal' or 'balanced', analgesia.

The mechanism underlying the use of multimodal analgesia is to use analgesics having a different mode of analgesic action, which allows the dose of the analgesics to be reduced and results in a lowered incidence of side effects. The basic goal of this strategy is a synergistic, or at least, additive, analgesic interaction between the combined drugs, here acetaminophen and nefopam.

The literature provides strong evidence of the efficacy of such a combination..In a review of preclinical and clinical studies, Girard et al., Nefopam analgesia and its role in multimodal analgesia: A review of preclinical and clinical studies. Clin Exp Pharmacol Physiol. 2016 Jan;43(1):3-12, it was found that a combination of nefopam and acetaminophen induced a more potent analgesia compared to acetaminophen or nefopam alone. Furthermore, the use of nefopam administered in combination with acetaminophen is advantageously since nefopam does hot have any of the known side-effects normally associated with opioids, that is, it does not bind to opioid receptors, does not induce respiratory depression, has no effect on platelet function and does not induce an anti-inflammatory effect.

However, there have been no studies finding that nefopam has a positive effect on the hepatoxiticity induced by acetaminophen. In fact earlier studies have indicated that nefopam exacerbate the hepatotoxicity of acetaminophen since high doses of these compounds, used concurrently, have given rise to hepatotoxicity in dogs, (SmPC nefopam June 2018). This study showed that 236mg/kg/day oral doses of acetaminophen and 24mg/kg/day doses of nefopam trigger potentiating of hepatotoxicity of acetaminophen (SmPC nefopam, 28/02/2012).

However, contrary to these studies, the inventors of the present invention have found that in addition to the advantageous effects of co-administration a combinations of analgesics, the combination of nefopam with acetaminophen reduces and/or even eliminates the hepatotoxicity effect of acetaminophen. Thus, the pharmaceutical composition according to the present invention makes it possible to safely administer acetaminophen at higher dosages.

Furthermore, both intentional and accidental acetaminophen over dosage is effectively prevented. Intentional overdose (attempted suicide) is the most common cause of acetaminophen toxicity in Great Britain, and unintentional overdose is the most frequent cause in the United States, representing almost 50% of the total intoxication cases.

The inventors of the present invention have found that in order for the protective mechanism of nefopam to be observed, it is advantageous that nefopam and acetaminophen are administered at a certain weight ratio in relation to each other. Said weight ratio is preferably such that for each 100 mg acetaminophen the composition comprises from 3 mg to 20 mg nefopam, preferably from 5 mg to 12 mg nefopam per 100 mg acetaminophen.

A person skilled in the art will understand that the dosages can be adjusted in dependence of each other, thus if the pharmaceutical composition for instance comprises 300 mg acetaminophen the composition may comprises from 9 mg nefopam to 60 mg nefopam, etc.

In one preferred embodiment the pharmaceutical composition according to the invention comprises 300 mg acetaminophen and from 17.65 to 35.29 mg nefopam, as such a dosage interval has proven to provide results relating to acetaminophen overdose treatment with NAC. Thus, said preferred composition comprises from 5.89 to 11.76 mg nefopam per 100 mg acetaminophen.

The pharmaceutical composition may in addition to the acetaminophen and nefopam comprise one or more excipient(s). Said excipients are preferably selected from the group consisting of granulating agents, diluents, solvents, glidants, surfactants, preservatives, solubilizers, emulsifiers, plasticizers and the like. The number of excipients that can be included in a composition is not limited.

Examples of diluents/fillers include, but not limited to, celluloses, cellulose acetate, microcrystalline cellulose, co-processed microcrystalline celluloses (such as various grades of Avicel), silicified microcrystalline cellulose, dextrates, dextrin, dextrose, fructose, glyceryl palmitostearate, hydrogenated vegetable oil, kaolin, lactitol, lactose, maltitol, mannitol, maltodextrin, maltose, pregelatinized starch, sodium chloride, sorbitol, starches, sucrose, glucose, trehalose, erythritol, fructose, calcium sulphate, dibasic calcium phosphate, talc and xylitol or a mixture of one or more of said diluents. However, in a preferred embodiment the diluents/filler is microcrystalline cellulose.

Suitable binders include, but are not limited to, celluloses such as microcrystalline cellulose, modified celluloses such as low substituted hydroxypropyl cellulose, hydroxypropyl cellulose, hydroxypropyl methylcellulose, hydroxyethylcellulose, hydroxyethyl methylcellulose, cellulose gum, xanthan gum, sugars (such as sucrose, glucose, amylose, maltodextrin, dextrose and the like), starches such as corn or potato starch, pregelatinized starches, polyvinyl alcohol-polyethylene glycol graft copolymer, copovidone, povidone, carbomers, polycarbophil, polyethylene oxide, polyethylene glycol or a combination of suitable binders. However, in a preferred embodiment the binder is povidone K90 and/or Starch.

Examples of disintegrants include, but not limited to starches, partially pregelatinized starches, sodium starch glycolate, pregelatinized starch, alginic acid, powdered cellulose, croscarmellose sodium, crospovidone, docusate sodium, guar gum, hydroxypropyl cellulose, low substituted hydroxypropyl cellulose, magnesium aluminum silicate, methylcellulose, sodium alginate or a combination of one or more disintegrants. However, in a preferred embodiment the disintegrant is croscarmellose sodium.

The invention also relates to a solid unit dosage of the pharmaceutical composition according to the invention. Said unit dosage is preferably a tablet or a capsule arranged for oral administration, but other administration forms are also contemplated within the scope of the present invention.

In a preferred embodiment the unit dosage comprises between 100 and 1000 mg acetaminophen, preferably between 200 and 750 mg acetaminophen and even more preferred between 300 and 600 mg acetaminophen.

The unit dosage furthermore comprises nefopam in the ratio defined by the present invention, e.g. if the unit dosage comprises 500 mg acetaminophen, the composition comprise from 15 mg to 100 mg nefopam, preferably from 25 mg to 60 mg nefopam, and even more preferred around 50 mg nefopam.

In a further preferred embodiment the unit dosage comprises 500 mg acetaminophen and from 29.42 mg nefopam to 58.82 mg nefopam. An alternative unit dosage comprises 325 mg acetaminophen and 20 mg nefopam

In this respect the median effective analgesic dose (median value and 95%'confidence interval) of nefopam and acetaminophen were 30 mg and 500 mg, respectively. Thus, a preferred oral unit dosage for management of pain which at the same time can be used for preventing and/or reducing hepatotoxicity induced by acetaminophen is as follows:

| Components | (mg) |
|---|---|
| Acetaminophen | 500 |
| Microcrystalline cellulose | 146.7 |
| Povidone K90 | 33 |
| Starch | 18.3 |
| Anhydrous citric acid | 1.5 |
| Nefopam HCL | 30 |
| Magnesium stearate | 4 |

### EXAMPLES

### Example 1: Toxicology of acetaminophen and/or nefopam

Liver function in rats is measured and the progression of liver damage is monitored to screen for liver disease in invasive and non-invasive manner. Alanine aminotransferase (ALAT) and aspartate aminotransferase (ASAT), two markers of hepatotoxicity was assayed using UV test according to IFCC recommendations.

The toxicology of acetaminophen, nefopam and a combination of acetaminophen and nefopam was initially evaluated according to the scheme in table 1:

**Table 1**

| **Group** | **Treatment** | **Dose level (mg/kg/day)** | **Main groups (rats)** |
|---|---|---|---|
| 1 | Vehicle | - | 10 Male, 10 Female |
| 2 | Paracetamol (P) | 505 for 4 days then 645 for 24 days | 10 Male, 10 Female |
| 3 | Nefopam (N) | 30 for 4 days then 38.6 for 24 days | 10 Male, 10 Female |
| 4 | Nefopam (N)/Paracetamol(P) | N at 18.5 / P at 308.6 for 28 days | 10 Male, 10 Female |
| 5 | Nefopam(N)/Paracetamol(P) | N at 30 / P at 505 for 4 days then N at 38.6 / P at 645 for 24 days | 10 Male, 11 Female |

Control animals (group 1) received the vehicle only (1% Tween 80 in 0.5% Methylcellulose in water).

The results are shown in table 2 and 3 below.

### Results after 4 weeks.

**Table 2 *p<0.05; **p<0.01 vs vehicle-treated group**

| **Group** | **males** | **ALAT (U/L)** | **ASAT (U/L)** | **Group** | **females** | **ALAT (U/L)** | **ASAT (U/L)** |
|---|---|---|---|---|---|---|---|
| **1** | Mean | 37 | 90 | **1** | Mean | 33 | 105 |
| | SEM | 3 | 3 | | SEM | 1 | 5 |
| | N | 20 | 20 | | N | 20 | 20 |
| **2** | Mean | 59 | 110 | **2** | Mean | 49 | 101 |
| | SEM | 4 | 6 | | SEM | 5 | 7 |
| | N | 10 ** | 10 * | | N | 10 ** | 10 |
| **3** | Mean | 35 | 83 | **3** | Mean | 31 | 96 |
| | SEM | 2 | 4 | | SEM | 1 | 6 |
| | N | 10 | 10 | | N | 9 | 10 |
| **4** | Mean | 52 | 102 | **4** | Mean | 35 | 91 |
| | SEM | 7 | 11 | | SEM | 3 | 5 |
| | N | 10 * | 10 | | N | 10 | 10 |
| **5** | Mean | 59 | 98 | **5** | Mean | 41 | 92 |
| | SEM | 2 | 3 | | SEM | 2 | 3 |
| | N | 19 ** | 20 | | N | 19 * | 19 |

As is evident from table 2, acetaminophen resulted in an increase in transaminase levels whereas nefopam by itself, had no effect on said levels.

When nefopam was combined with acetaminophen, nefopam did not exacerbate the level increase of transaminase due to acetaminophen, after 4 weeks of treatment. Moreover there was no significant change of AST levels (vs. vehicle) when this combination was tested.

### Results after 6 weeks.

**Table 3 *p<0.05; **p<0.01 vs vehicle-treated group**

| **Group** | **males** | **ALAT (U/L)** | **ASAT (U/L)** | **Group** | **females** | **ALAT (U/L)** | **ASAT (U/L)** |
|---|---|---|---|---|---|---|---|
| **1** | Mean | 31 | 86 | **1** | Mean | 29 | 78 |
| | SEM | 2 | 9 | | SEM | 2 | 3 |
| | N | 10 | 10 | | N | 9 | 10 |
| **5** | Mean | 34 | 77 | **5** | Mean | 28 | 74 |
| | SEM | 3 | 5 | | SEM | 1 | 2 |
| | N | 10 | 10 | | N | 9 | 9 |

As is evident from table 3, levels of both ALAT and ASAT after treatment of rats with the nefopam/acetaminophen combination were not different from those of the vehicle-treated group.

### Conclusion

Acetaminophen increases transaminase levels while nefopam has no effect. Moreover, nefopam seems to reduce the increase of transaminase caused by acetaminophen.

### Example 2: Assessment of acute hepatotoxicity.

Investigation of the preventive effect of nefopam on acetaminophen-induced acute liver injury with two endpoints after 24 hours:
- assessment of serum biomarkers of liver injury: alanine aminotransferase, and aspartate aminotransferase,
- histopathological assessment of liver lesions.

192 male mice CD-1 (Janvier, France) (12 mice per group) with an age of 7-10 weeks were used. They received the products intraperitoneally (i.p.).

*Acclimatization:* Animals were housed a minimum of one week before the study began, in the area where the study took place in order to habituate the animals to the experimental conditions.

*Housing:* Animals were housed 8-10 per cage of standard dimension with size (42.5 x 26.6 x 15.5 cm) under controlled environmental conditions (22°C; 55% humidity) and kept under a 12/12h light/dark cycle.

*Feeding:* Animals were fed ad libitum with a standard diet (Safe A04, Epinay/Orge, France) from the day of arrival in the experimentation site.

*Drinking water:* Filtered tap water was made available ad libitum.

*Animal care:* Experiments were carried out in accordance with the Committee for Research and Ethical Issues of the IASP (Zimmermann, 1983). All efforts were made to minimize suffering of the animals, in accordance with the National Institute of Health Guidelines for the Care and Use of Laboratory Animals. Animals were euthanized promptly when their injuries exceeded the nails biting.

*Determination of serum biomarkers:* Liver injury was evaluated by measuring the serum levels of alanine aminotransferase (ALAT), and aspartate aminotransferase (ASAT) using a spectrophotometric assay method.

*Histopathological assessment of liver lesions:* The histopathological lesions in the liver were evaluated as follows: The collected tissues were processed with standard procedures and embedded in paraffin. The embedded tissues were sectioned into 4 µm thick sections using a microtome. These tissue sections were stained with hematoxylin and eosin (H&E) according to standard techniques and examined by a light microscope.

The severity of necrotic lesions in liver parenchyma was determined.
- Grade 0:: No pathological change
- Grade 1:: Presence of degenerated hepatocytes with only rare foci of necrosis
- Grade 2:: Small area of mild centrilobular necrosis around the central vein
- Grade 3:: Area of mild centrilobular necrosis severer than Grade 2
- Grade 4:: Centrilobular necrosis severer than Grade 3

*Protocol:* 24h after treatments, animals were euthanized. Plasma and liver were sampled.

*Statistical analysis:* Results were expressed as means ± standard error of mean (S.E.M.).

The statistics software used was GraphPad Prism 7.

The significance level was set at p < 0.05.

### Dosage regime:

| | | |
|---|---|---|
| 300 mg/kg acetaminophen | + | Vehicle |
| 300 mg/kg acetaminophen | + | 1.1 mg/kg nefopam |
| 300 mg/kg acetaminophen | + | 2.2 mg/kg nefopam |
| 300 mg/kg acetaminophen | + | 4.4 mg/kg nefopam |
| 300 mg/kg acetaminophen | + | 8.82 mg/kg nefopam |
| 300 mg/kg acetaminophen | + | 17.65 mg/kg nefopam |
| 300 mg/kg acetaminophen | + | 35.29 mg/kg nefopam |
| 300 mg/kg acetaminophen | + | 200 mg/kg NAC |
| Vehicle | + | Vehicle |
| Vehicle | + | 1.1 mg/kg nefopam |
| Vehicle | + | 2.2 mg/kg nefopam |
| Vehicle | + | 4.4 mg/kg nefopam |
| Vehicle | + | 8.82 mg/kg nefopam |
| Vehicle | + | 17.65 mg/kg nefopam |
| Vehicle | + | 35.29 mg/kg nefopam |
| Vehicle | + | 200 mg/kg NAC |

The vehicle for paracetamol consisted of 0.9% NaCl saline solution weakly heated for paracetamol dilution, and the vehicle for nefopam consisted of 0.9% NaCl saline solution.

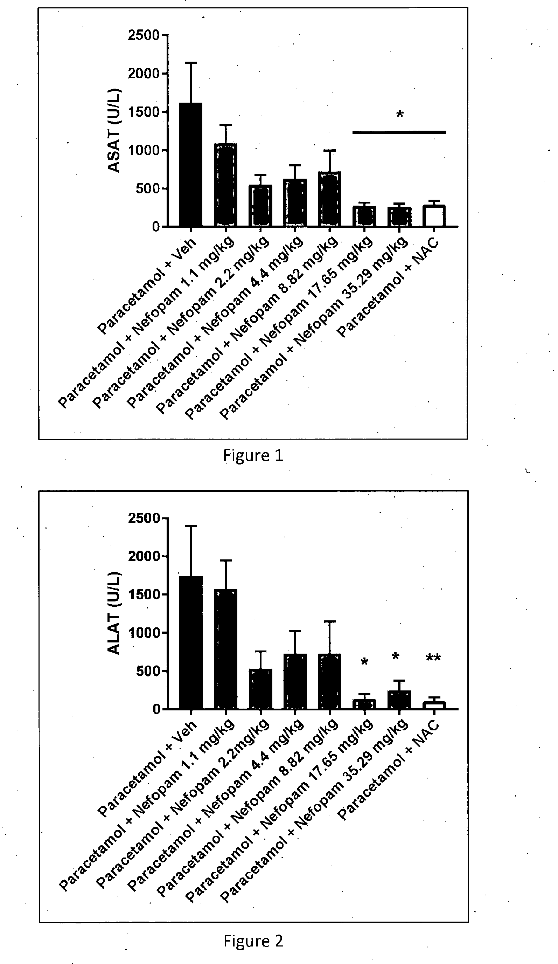

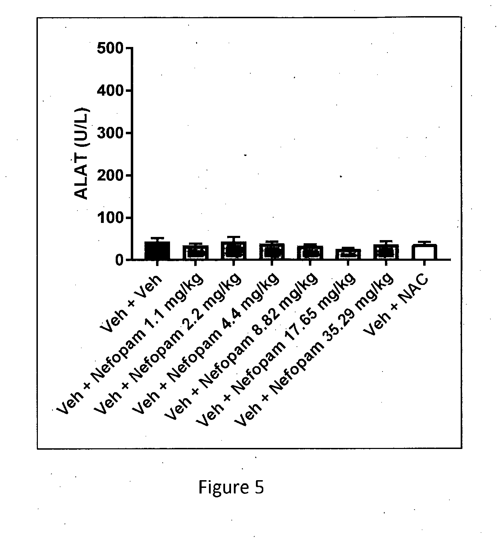

The individual results are shown in table 4 - 19, and in figure 1 to 5. The figures include the means + S.E.M.
**300 mg/kg Acetaminophen + Vehicle**

**Table 4**

| | | | | | | | | | | | | | | |
|---|---|---|---|---|---|---|---|---|---|---|---|---|---|---|
| **ASAT (U/L)** | 4302 | 3291 | 743 | 311 | 5403 | 299 | 378 | 484 | 448 | 131 | 1076 | 214 | 4936 | 708 |
| **ALAT (U/L)** | 1221 | 3379 | 1308 | 122 | 8055 | 33 | 214 | 51 | 49 | 36 | 1851 | 259 | 5979 | 1775 |
| **Score** | 4 | 3 | 4 | 0 | 4 | 0 | 2 | 0 | 0 | 0 | 3 | 3 | 4 | 2 |

**300 mg/kg Acetaminophen + 1.1 mg/kg nefopam**

**Table 5**

| | | | | | | | | | | | | | | |
|---|---|---|---|---|---|---|---|---|---|---|---|---|---|---|
| **ASAT (U/L)** | 2020 | 1479 | 1067 | 1470 | 3600 | 566 | 150 | 1137 | 562 | 776 | 232 | 143 | 942 | 1098 |
| **ALAT (U/L)** | 4060 | 1206 | 1340 | 1730 | 4619 | 323 | 116' | 2965 | 1328 | 688 | 134 | 293 | 1660 | 1528 |
| **Score** | 3 | 4 | 3 | 3 | 3 | 0 | 0 | 3 | 4 | 2 | 1 | 3 | 3 | 3 |

**300 mg/kg Acetaminophen + 2.2 mg/kg nefopam**

**Table 6**

| | | | | | | | | | | | | | | |
|---|---|---|---|---|---|---|---|---|---|---|---|---|---|---|
| **ASAT (U/L)** | 373 | 206 | 307 | 362 | 721 | 100 | 345 | 289 | 134 | 1398 | 787 | 1679 | 817 | 235 |
| **ALAT (U/L)** | 101 | 40 | 29 | 194 | 64 | 18 | 40 | 56 | 24 | 2209 | 854 , | 2460 | 1043 | 349 |
| **Score** | 0 | 0 | 0 | 0 | 0 | 0 | 0 | 0 | 0 | 4 | 1 | 4 | 3 | 3 |

**300 mg/kg Acetaminophen + 4.4 mg/kg nefopam**

**Table 7**

| | | | | | | | | | | | | | | |
|---|---|---|---|---|---|---|---|---|---|---|---|---|---|---|
| **ASAT (U/L)** | 190 | 642 | 634 | 191 | 405 | 446 | 163 | 290 | 481 | 128 | 2530 | 1679 | 416 | 558 |
| **ALAT (U/L)** | 68 | 51 | 363 | 61 | 43 | 48 | 41 | 44 | 610 | 43 | 3210 | 3081 | 779 | 1742 |
| **Score** | 0 | 0 | 1 | 0 | 1 | 0 | 0 | 1 | 3 | 1 | 3 | 4 | 2 | 3 |

**300 mg/kg Acetaminophen + 8.82 mg/kg nefopam**

**Table 8**

| | | | | | | | | | | | | | | |
|---|---|---|---|---|---|---|---|---|---|---|---|---|---|---|
| **ASAT (U/L)** | 2783 | 206 | 152 | 267 | 479 | 719 | 188 | 554 | 265 | 217 | 461 | 107 | 3716 | 579 |
| **ALAT (U/L)** | 2512 | 23 | 60 | 27 | 41 | 72 | 19 | 390 | 49 | 41 | 41 | 29 | 5618 | 1277 |
| **Score** | 4 | 0 | 2 | 0 | 0 | 0 | 0 | 3 | 0 | 0 | 0 | 0 | 3 | 2 |

**300 mg/kg Acetaminophen + 17.65 mg/kg nefopam**

**Table 9**

| | | | | | | | | | | | | | | |
|---|---|---|---|---|---|---|---|---|---|---|---|---|---|---|
| **ASAT (U/L)** | 425 | 153 | 230 | 107 | 396 | 719 | 235 | 253 | 352 | 103 | 324 | 148 | 281 | 98 |
| **ALAT (U/L)** | 233 | 18 | 31 | 27 | 62 | 119 | 45 | 43 | 50 | 23 | 1024 | 75 | 35 | 40 |
| **Score** | 0 | 0 | 0 | 0 | 0 | 0 | 0 | 0 | 0 | 0 | 3 | 0 | 0 | 0 |

**300 mg/kg Acetaminophen + 35.29 mg/kg nefopam**

**Table 10**

| | | | | | | | | | | | | | | |
|---|---|---|---|---|---|---|---|---|---|---|---|---|---|---|
| **ASAT (U/L)** | 331 | 264 | 198 | 394 | 404 | 255 | 370 | 240 | 162 | 131 | 63 | 86 | 659 | 167 |
| **ALAT (U/L)** | 545 | 228 | 39 | 34 | 240 | 21 | 295 | 21 | 27 | 28 | 14 | 24 | 1862 | 71 |
| **Score** | 0 | 0 | 0 | 0 | 0 | 0 | 2 | 0 | 0 | 0 | 0 | 0 | 3 | 0 |

**300 mg/kg Acetaminophen + 200 mg/kg NAC**

**Table 11**

| | | | | | | | | | | | | | | |
|---|---|---|---|---|---|---|---|---|---|---|---|---|---|---|
| **ASAT (U/L)** | 231 | 168 | 190 | 96 | 109 | 371 | 447 | 621 | 119 | 128 | 682 | 276 | 359 | 253 |
| **ALAT (U/L)** | 26 | 22 | 34 | 40 | 32 | 51 | 170 | 36 | 26 | 23 | 152 | 48 | 745 | 28 |
| **Score** | 1 | 0 | 0 | 0 | 0 | 0 | 0 | 0 | 0 | 0 | 0 | 0 | 1 | 0 |

**Vehicle + Vehicle**

**Table 12**

| | | | | | | | | | | | | |
|---|---|---|---|---|---|---|---|---|---|---|---|---|
| **ASAT (U/L)** | 198 | 112 | 180 | 205 | 230 | 504 | 497 | 322 | 410 | 383 | 233 | 307 |
| **ALAT (U/L)** | 26 | 18 | 39 | 27 | 29 | 32 | 44 | 33 | 50 | 57 | 30 | 135 |
| **Score** | 0 | 0 | 0 | 0 | 0 | 0 | 0 | 0 | 0 | 0 | 0 | 0 |

**Vehicle + 1.1 mg/kg nefopam**

**Table 13**

| | | | | | | | | | | | | |
|---|---|---|---|---|---|---|---|---|---|---|---|---|
| **ASAT (U/L)** | 98 | 152 | 193 | 181 | 136 | 426 | 110 | 97 | 210 | 202 | 266 | 180 |
| **ALAT (U/L)** | 17 | 23 | 31 | 45 | 19 | 55 | 49 | 27 | 28 | 29 | 55 | 51 |
| **Score** | 0 | 0 | 0 | 0 | 0 | 0 | 0 | 0 | 0 | 0 | 0 | 0 |

**Vehicle + 2.2 mg/kg nefopam**

**Table 14**

| | | | | | | | | | | | | |
|---|---|---|---|---|---|---|---|---|---|---|---|---|
| **ASAT (U/L)** | 342 | 325 | 119 | 118 | 221 | 129 | 269 | 785 | 148 | 111 | 395 | 59 |
| **ALAT (U/L)** | 30 | 152 | 24 | 27 | 35 | 30 | 60 | 57 | 25 | 19 | 52 | 19 |
| **Score** | 0 | 0 | 0 | 0 | 0. | 0 | 0 | 0 | 0 | 0 | 0 | 0 |

**Vehicle + 4.4 mg/kg nefopam**

**Table 15**

| | | | | | | | | | | | | |
|---|---|---|---|---|---|---|---|---|---|---|---|---|
| **ASAT (U/L)** | 541 | 151 | 79 | 89 | 163 | 531 | 71 | 466 | 183 | 128 | 111 | 93 |
| **ALAT (U/L)** | 55 | 51 | 26 | 28 | 42 | 43 | 24 | 71 | 23 | 25 | 22 | 43 |
| **Score** | 0 | 0 | 0 | 0 | 0 | 0 | 0 | 0 | 0 | 0 | 0 | 0 |

**Vehicle + 8.82 mg/kg nefopam**

**Table 16**

| | | | | | | | | | | | | |
|---|---|---|---|---|---|---|---|---|---|---|---|---|
| **ASAT (U/L)** | 99 | 175 | 154 | 272 | 447 | 576 | 248 | 149 | 163 | 76 | 334 | 235 |
| **ALAT (U/L)** | 39 | 26 | 33 | 18 | 47 | 52 | 36 | 29 | 33 | 25 | 50 | 35 |
| **Score** | 0 | 0 | 0 | 0 | 0 | 0 | 0 | 0 | 0 | 0 | 0 | 0 |

**Vehicle + 17.65 mg/kg nefopam**

**Table 17**

| | | | | | | | | | | | | |
|---|---|---|---|---|---|---|---|---|---|---|---|---|
| **ASAT (U/L)** | 186 | 100 | 145 | 202 | 183 | 360 | 133 | 167 | 158 | 157 | 205 | 143 |
| **ALAT (U/L)** | 31 | 25 | 32 | 23 | 27 | 35 | 27 | 26 | 25 | 15 | 40 | 24 |
| **Score** | 0 | 0 | 0 | 0 | 0 | 0 | 0 | 0 | 0 | 0 | 0 | 0 |

**Vehicle + 35.29 mg/kg nefopam**

**Table 18**

| | | | | | | | | | | | | |
|---|---|---|---|---|---|---|---|---|---|---|---|---|
| **ASAT (U/L)** | 147 | 359 | 201 | 790 | 223 | 196 | 342 | 240 | 412 | 125 | 272 | 200 |
| **ALAT (U/L)** | 18 | 35 | 26 | 114 | 22 | 29 | 35 | 21 | 46 | 32 | 44 | 25 |
| **Score** | 0 | 0 | 0 | 0 | 0 | 0 | 0 | 0 | 0 | 0 | 0 | 0 |

**Vehicle + 200 mg/kg NAC**

**Table 19**

| | | | | | | | | | | | | |
|---|---|---|---|---|---|---|---|---|---|---|---|---|
| **ASAT (U/L)** | 292 | 320 | 160 | 360 | 107 | 283 | 723 | 170 | 183 | 101 | 114 | 184 |
| **ALAT (U/L)** | 41 | 58 | 31 | 28 | 14 | 29 | 71 | 51 | 41 | 26 | 27 | 30 |
| **Score** | 0 | 0 | 0 | 0 | 0 | 0 | 0 | 0 | 0 | 0 | 0 | 0 |

### Conclusion:

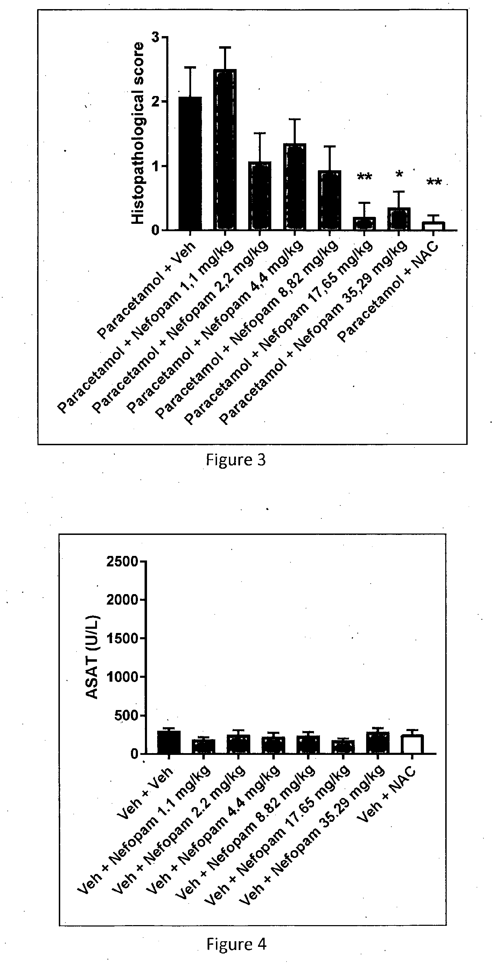

Acetaminophen (300 mg/kg, i.p.) treatment (acetaminophen + Veh group) induced in mice an elevation of ASAT (Figure 1), ALAT (Figure 2) and induced histological liver lesions (Figure 3). N-acetyl-cysteine, coadministered with acetaminophen (acetaminophen + NAC group) reduced acetaminophen-induced hepatotoxicity (Figures 1 - 3).

Co-administered with acetaminophen, nefopam, at doses of 17.65 and 35.29 mg/kg (i.p.), significantly reduced ASAT and ALAT elevations (Figures 1 and 2) and histological score (Figure 3). Moreover, treatment of nefopam with vehicle (without acetaminophen) did not influence ASAT and ALAT plasma concentration (Figures 4 and 5) and induced no histological liver lesion (histological score = 0 for all mice).

These results demonstrated for the first time that nefopam, co-administered with acetaminophen, is able to abolish acetaminophen-induced hepatotoxicity in mice.

These results, with those showing that nefopam has a synergic effect on nociception (Girard et al., 2011; Van Elstraete and Sitbon, 2013; Cabañero and Maldonado, 2021), suggest that the combination of nefopam and acetaminophen can increase the benefit-to-risk ratio of acetaminophen.

Thus, the inventors have found that nefopam in dosages from 5 mg/kg nefopam to 12 mg/kg nefopam per 100 mg/kg acetaminophen, i.e. 15 mg/kg nefopam to 36 mg/kg nefopam per 300 mg/kg acetaminophen effectively can be used for preventing and/or reducing hepatotoxicity induced by acetaminophen.

In one preferred embodiment, the pharmaceutical composition according to the invention comprises 300 mg acetaminophen and from 17.65 to 35.29 mg nefopam, as such a dosage interval has proven to provide results relating to acetaminophen overdose treatment with NAC.

## Claims

1. A pharmaceutical composition for use in preventing and/or reducing hepatotoxicity induced by acetaminophen, and wherein said pharmaceutical composition comprises a combination of acetaminophen and nefopam.

2. A pharmaceutical composition according to claim 1, wherein the pharmaceutical composition comprises from 3 mg to 20 mg nefopam per 100 mg acetaminophen.

3. A pharmaceutical composition according to any of the claims 1 or 2, wherein the pharmaceutical composition comprises from 5 mg to 12 mg nefopam per 100 mg acetaminophen,

4. A pharmaceutical composition according to any of the preceding claims, wherein the pharmaceutical composition comprises between 100 and 1000 mg acetaminophen, preferably between 200 and 750 mg acetaminophen and even more preferred between 300 and 600 mg acetaminophen.

5. A pharmaceutical composition according to any of the preceding claims, wherein pharmaceutical composition further comprises at least one excipient.

6. A pharmaceutical composition according to claim 5, wherein the least one excipient is selected from the group consisting of granulating agents, diluents, solvents, glidants, surfactants, preservatives, solubilizers, emulsifiers, plasticizers and the like.

7. A solid unit dosage comprising the pharmaceutical composition according to any of the claims 1 - 6.

8. A solid unit dosage according to claim 7, wherein the unit dosage is in the form of a tablet or a capsule.

9. A liquid unit dosage comprising the pharmaceutical composition according to any of the claims 1 - 6.

10. An analgesia composition consisting of the pharmaceutical composition according to any of the claims 1 - 6 or the unit dosage according to any of the claims 7 - 9, wherein the analgesic effect of acetaminophen is improved by using a multimodal approach.
